Europäisches Patentamt

European Patent Office

Office européen des brevets

(19)

(11) Veröffentlichungsnummer: **0 479 066 B1**

(12) **EUROPÄISCHE PATENTSCHRIFT**

(45) Veröffentlichungstag der Patentschrift: **07.12.94**

(51) Int. Cl.5: **A61K 9/08**, A61K 31/07

(21) Anmeldenummer: **91116027.3**

(22) Anmeldetag: **20.09.91**

(54) **Verfahren zur Herstellung von stabilen injizierbaren beta-Carotin-Solubilisaten.**

(30) Priorität: **02.10.90 DE 4031094**

(43) Veröffentlichungstag der Anmeldung:
**08.04.92 Patentblatt 92/15**

(45) Bekanntmachung des Hinweises auf die
Patenterteilung:
**07.12.94 Patentblatt 94/49**

(84) Benannte Vertragsstaaten:
**BE CH DE DK ES FR GB IT LI NL**

(56) Entgegenhaltungen:
**EP-A- 0 055 817**
**WO-A-89/07929**
**FR-A- 2 300 554**
**US-A- 2 976 024**
**US-A- 3 932 634**

(73) Patentinhaber: **BASF Aktiengesellschaft
Carl-Bosch-Strasse 38
D-67063 Ludwigshafen (DE)**

(72) Erfinder: **End, Lutz, Dr.
Tannhaeuser Ring 103-105
W-6800 Mannheim 24 (DE)**
Erfinder: **Horn, Dieter, Dr.
Schroederstrasse 69
W-6900 Heidelberg (DE)**
Erfinder: **Lueddecke, Erik, Dr.
Thomas-Mann-Strasse 27
W-6704 Mutterstadt (DE)**
Erfinder: **Schneider, Joachim U., Dr.
Plauserstrasse 17
W-6719 Weisenheim (DE)**
Erfinder: **Hoppe, Peter Paul, Dr.
Am Hauenstein 13
W-6706 Wachenheim (DE)**
Erfinder: **Rensmann, Friedrich-Wilhelm, Dr.
Philipp-Fauth-Strasse 6
W-6702 Bad Duerkheim (DE)**

EP 0 479 066 B1

## Beschreibung

Die Erfindung betrifft die kontinuierliche Herstellung von $\beta$-Carotin-Solubilisaten durch ein Mischkammerverfahren.

Nach einem in EP-B-055 817 beschriebenen Batch-Verfahren gelingt die Herstellung einer wäßrigen $\beta$-Carotin-Injektionslösung unter Verwendung eines Emulgators. Dazu wird der Emulgator auf 170 bis 180°C erhitzt und $\beta$-Carotin in die Schmelze eingetragen, wobei das $\beta$-Carotin unter teilweiser Isomerisierung in Lösung geht. Nach einer Verweilzeit von ca. 6 Minuten wird die Schmelze auf 95°C abgekühlt und sodann Wasser zunächst tropfenweise eingetragen und das Produkt schließlich auf einen Wirkstoffgehalt von 4,5 % mit Wasser eingestellt. Der Emulgator-Gehalt beträgt in der Regel ca. 25 %.

Wegen der Schwerlöslichkeit des $\beta$-Carotins muß das Solubilisat eine ausreichende Stabilität gegen Rekristallisation im Lagertest aufweisen, die durch ein optimales Isomerenverhältnis gewährleistet wird. Die Herstellung eines spezifikationsgerechten Produktes setzt daher die gezielte Steuerung der Isomerisierungsreaktion voraus. Dies ist in dem beschriebenen Batch-Prozeß nur schwer erreichbar.

Es bestand daher die Aufgabe, ein kontinuierliches Herstellverfahren mit gezielter Steuerung der Isomerisierungsreaktion vorzuschlagen.

Diese Aufgabe wurde erfindungsgemäß gelöst mit einem Verfahren zur kontinuierlichen Herstellung von $\beta$-Carotin-Solubilisaten (durch kurzzeitiges Erhitzen von $\beta$-Carotin zusammen mit einem Emulgator bis zur Lösung, schnelle Abkühlung der homogenen Lösung auf unter 100°C durch Zugabe von Wasser und anschließende Einstellung der gewünschten Endkonzentration an $\beta$-Carotin) indem man eine auf 20 bis 80°C vorgewärmte Suspension von 1 bis 40 Gew.-% $\beta$-Carotin in einem Emulgator durch eine in einem Wärmeträgeröl befindliche Heizschlange pumpt, wobei die Temperatur in dem Solubilisierungsgemisch 120 bis 180°C und die Verweilzeit 10 bis 300 Sekunden beträgt und die homogene Lösung in einer Mischkammer, ggf unter einem auf 10 - 50 bar erhöhten Druck mit solchen Mengen Wasser von 10 bis 80°C turbulent vermischt, daß eine schnelle Abkühlung der homogenen Lösung auf unter 100°C erfolgt und ein Solubilisat mit 0,5 bis 6 Gew.-% $\beta$-Carotin entsteht, das gegebenenfalls auf die gewünschte Endkonzentration weiter verdünnt wird.

Die Mischkammer kann in Form eines T ausgebildet sein, in der das Wasser auf die $\beta$-Carotinlösung in einem Winkel von ungefähr 180° auftritt. Ausschlaggebend für die Formgebung der Mischkammer ist, daß eine turbulente Durchmischung der homogenen Lösung und der wäßrigen Phase gewährleistet ist.

Als Emulgatoren kommen an sich bekannte nicht-ionogene Emulgatoren mit einem HLB-Wert (vgl. H.P. Fiedler, Lexikon der Pharmazie, Kosmetik und angrenzenden Gebiete, 1971, Seiten 263 - 270, besonders Seiten 267-269) von 12 bis 16 in Betracht, insbesondere ethoxylierte Triglyceride von Fettsäuren mit 12 bis 18 Kohlenstoffatomen, die 20 bis 60 Oxethyleneinheiten enthalten oder ethoxylierte Sorbitanfettsäureester mit etwa 20 Oxethyleneinheiten oder ethoxylierte Monohydroxifettsäuren mit 14 bis 17 Oxethyleneinheiten, wie sie in der DE-OS 29 11 241 beschrieben sind. Derartige Emulgatoren heißen auch Solubilisatoren, weil sie sich in Wasser lösen und dadurch als Lösungsvermittler für lipophile Substanzen wirken, indem diese in micellarer Lösung gehalten werden. Mizellare Lösungen zeichnen sich durch Transparenz und Klarheit aus. Sie können charakterisiert werden durch die Angabe der Teilchengröße der Mizellen, bestimmt durch quasielastische Lichtstreuung. Entsprechende Durchmesser liegen zwischen 10 und 100 nm, je nach verwendetem Solubilisator und Wirkstoffgehalt.

Als besonders geeignete nicht-ionogene Emulgatoren seien beispielsweise genannt: Glycerinpolyoxethylenglykolricinoleat, Glycerinpolyoxethylenglykoloxystearat, Polyoxethylen(20)-sorbitanmonooleat, Polyoxethylen(20)sorbitanmonostearat und Monohydroxystearinsäure mit 15 Oxethyleneinheiten.

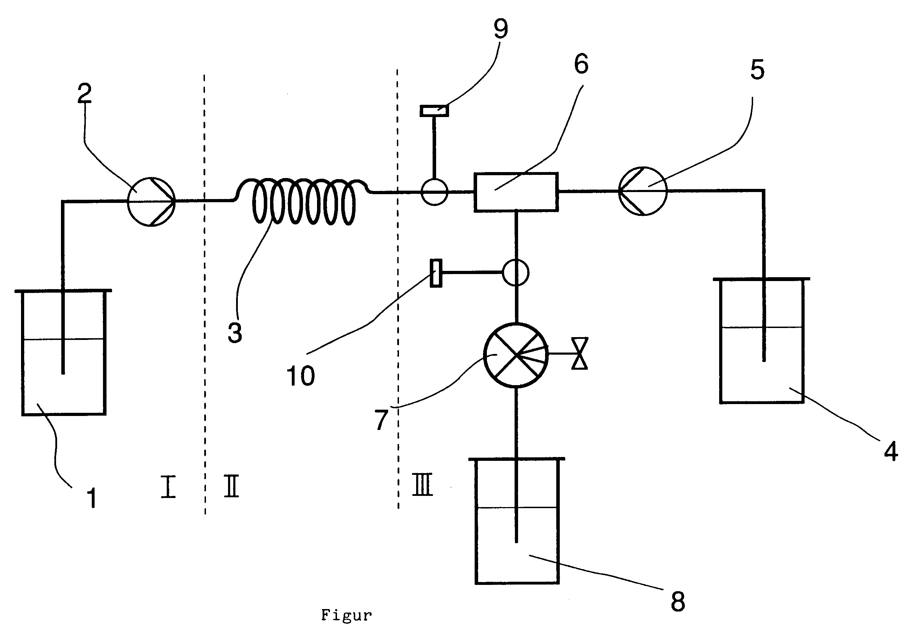

Bei der Herstellung der Solubilisate führt man das erfindungsgemäße Verfahren beispielsweise mit einer Apparatur, wie sie in Fig. 1 schematisch dargestellt ist, wie folgt durch:
Die Apparatur gliedert sich in die Teile I, II und III. Teil II ist der Hochtemperaturabschnitt, während in den Teilen I und III die Temperaturen weniger als 80°C betragen.

Im Gefäß (1) wird eine Suspension von $\beta$-Carotin in dem ausgewählten Emulgator in einer Konzentration von 1 - 40 Gew.-%, bevorzugt 10 - 30 %, gegebenenfalls unter Zusatz von 0,1 bis 10 % Antioxidantien, vorgelegt. Die Temperatur der Suspension wird auf 20 bis 80°C eingestellt, bevorzugt auf 60 - 70°C. Über die Pumpe (2) wird die Wirkstoff-Suspension kontinuierlich der Heizschlange (3) zugeführt. Diese befindet sich in einem geheizten Wärmeträgeröl. Die Förderleistung der Pumpe (2), die Länge der Heizschlange (3) und die Temperatur des Wärmeträgeröls werden so aufeinander abgestimmt, daß die Verweilzeit der Suspension in der Heizschlange zwischen 10 sek und 300 sek (bevorzugt 60 sek) bei einer an der Meßstelle (9) gemessenen Temperatur von 120 bis 180°C beträgt. In dieser Verweilzeit löst sich das $\beta$-Carotin unter gleichzeitiger Isomerisierung in dem Solubilisator auf. Nach Austritt aus der Heizschlange wird die jetzt vorliegende Lösung in der Mischkammer (6) mit der durch die Pumpe (5) aus dem Behälter (4) ebenfalls in die Mischkammer geförderte Wasserphase turbulent gemischt. In der Mischkammer bildet sich die mizellare $\beta$-Carotin-Lösung aus. Die Temperatur der Wasserphase liegt zwischen 10 und 80°C, bevorzugt bei 25°C. Die Wasserphase kann ein Konservierungsmittel, beispielsweise Benzylalkohol, in Konzentrationen zwischen 0,5 und 5 %, bezogen auf das Endprodukt enthalten. Die Temperatur des Solubilisats nach der Mischkammer wird an der Meßstelle (10) gemessen, sie liegt zwischen 40 und 80°C. Über den auf 10 - 50 bar eingestellten Druckbegrenzer (7) wird das Produkt in den Behälter (8) ausgetragen. Der Wirkstoffgehalt ergibt sich aus den jeweils gewählten Konzentrationen und Mengenströmen, er liegt in der Regel zwischen 0,5 und 6 %, bevorzugt bei 4 %.

Als übliche Antioxidantien, die bei dem erfindungsgemäßen Verfahren mitverwendet werden können, seien beispielsweise genannt: Butylhydroxitoluol, Butylhydroxianisol und d, l-$\alpha$-Tocopherol. Die Antioxidantien verwendet man im allgemeinen in Mengen von 10 bis 20 Gew.-%, bezogen auf eingesetztes $\beta$-Carotin.

Ferner verwendet man zweckmäßigerweise an sich bekannte und zugelassene Konservierungsmittel, wie $\beta$-Phenyl-ethylalkohol, $\beta$-Phenoxy-ethylalkohol und Benzylalkohol, insbesondere Benzylalkohol.

Mit dem erfindungsgemäßen Verfahren gelingt es, $\beta$-Carotinsolubilisateherzustellen, die hervorragend stabile Injektionslösungen ergeben. Die injizierbaren $\beta$-Carotinsolubilisate haben eine höhere Bioverfügbarkeit als oral verabreichtes $\beta$-Carotin. Die höhere Bioverfügbarkeit ist gekennzeichnet durch maximale $\beta$-Carotin-Blutspiegel, die z.B. bei Pferden, Rindern und Schweinen um 1 - 2 Zehnerpotenzen höher liegen als nach oraler Verabreichung. Durch Injektion von $\beta$-Carotinsolubilisaten kann bei landwirtschaftlichen Nutztieren wie Pferden, Rindern, Schweinen und Kaninchen die Fruchtbarkeit verbessert werden, indem der Prozentsatz trächtiger Tiere erhöht wird und mehr Jungtiere geboren werden.

Durch Wahl der Lösungstemperatur und der Verweilzeit kann bei dem erfindungsgemäßen Verfahren die thermische Isomerisierung gezielt gesteuert und somit ein Isomerenverhältnis eingestellt werden, das für Lagerstabilität und biologische Wirksamkeit optimal ist.

Beispiel

In einer beheizten Vorlage wurde bei einer Temperatur von 70°C eine Suspension von X g $\beta$-Carotin (vgl. Tabelle) in 250 g 13-Hydroxystearinsäureethoxylat und 10 g Butylhydroxytoluol vorgelegt. Diese Suspension wurde mittels Hochdruckpumpe (2) bei einem Durchsatz von 2 l/h der in ein Ölbad eingetauchten Heizschlange zugeführt. Bei einem inneren Durchmesser von 2 mm und einer Länge von 3 m, 6 m bzw. 12 m wurden bei Wärmeträgeröl-Temperaturen von ca. 160°C Verweilzeiten von 17, 34 bzw. 68 Sekunden eingestellt. Diese Zeiten reichten in allen Fällen aus, um das $\beta$-Carotin in dem Emulgator zu lösen. Nach den genannten Verweilzeiten in der Heizschlange trat die $\beta$-Carotin-Lösung in eine T-förmige Mischkammer ein, in der unter einem Mischungswinkel von 180° über die Hochdruckpumpe (5) 690 g VE-Wasser bei einem Durchsatz von 5 l/h turbulent zugemischt wurden. Bei einem Druck von 25 bar wurde das Produkt über ein Druckbegrenzer-Ventil ausgetragen. In allen Fällen wurde eine dunkelrot gefärbte, mizellare $\beta$-Carotin-Lösung erhalten. Die Produkttemperatur lag bei 60°C. Der Wirkstoffgehalt, lag je nach Fahrweise zwischen 4,3 und 5,5 %. Durch quasi-elastische Lichtstreuung wurden bei den Solubilisaten Mizellendurchmesser von 20 - 30 nm gemessen.

Alle Versuchsparameter und Meßdaten, einschließlich der ermittelten Isomerenverhältnisse, sind in der Tabelle dargestellt.

Tabelle

| Vers.-Nr. | $\beta$-Carotin-Einwaage | Heizschlange | | Temperaturen | |
| | | Länge | Verweilzeit | Lösung $T_L$ | Solubilisat $T_S$ |
| | [g] | [m] | [sec] | [°C] | [°C] |
| --- | --- | --- | --- | --- | --- |
| 1 | 50 | 3 | 17 | 158 | 58 |
| 2 | 50 | 12 | 68 | 153–160 | 57 |
| 3 | 42,5 | 12 | 68 | 150 | 60 |
| 4 | 42,5 | 3 | 17 | 159 | 60 |
| 5 | 42,5 | 6 | 34 | 169 | 61 |
| 6 | 50 | 6 | 34 | 162 | 64 |

EP 0 479 066 B1

Tabelle (Forts.)

| Vers.-Nr. | β-Carotin-gehalt [Gew.-%] | Mizell-größe [nm] | pH | Charakterisierung des Solubilisats Isomerengehalt | | |
| --- | --- | --- | --- | --- | --- | --- |
| | | | | all-trans [%*)] | 13-cis [%*)] | 9-cis [%*)] |
| 1 | 4,9 | 30±25 % | 7,0 | 66,4 | 18,1 | 4,1 |
| 2 | 4,5 | 21±30 | 6,9 | 36,9 | 21,7 | 22,5 |
| 3 | 4,3 | 20±22 | 7,0 | 38,0 | 20,7 | 21,9 |
| 4 | 4,5 | 20±22 | 6,7 | 56,6 | 23,2 | 5,3 |
| 5 | 4,6 | 21±20 | 7,0 | 50,6 | 17,5 | 16,4 |
| 6 | 5,5 | 22±28 | 6,7 | wie Versuch Nr. 5 | | |

*) Flächen-% HPLC bei 450 nm

## Patentansprüche

1. Verfahren zur kontinuierlichen Herstellung von β-Carotin-Solubilisaten durch kurzzeitiges Erhitzen von β-Carotin zusammen mit einem Emulgator bis zur Lösung, schnelle Abkühlung der homogenen Lösung auf unter 100°C durch Zugabe von Wasser und anschließende Einstellung der gewünschten Endkonzentrationen an β-Carotin, dadurch gekennzeichnet, daß man eine auf 20 bis 80°C vorgewärmte Suspension von 1 bis 40 Gew.-% β-Carotin in einem Emulgator durch eine in einem Wärmeträgeröl befindliche Heizschlange pumpt, wobei die Temperatur in dem Solubilisierungsgemisch 120 bis 180°C und die Verweilzeit 10 bis 300 Sekunden beträgt, und die homogene Lösung in einer Mischkammer mit solchen Mengen Wasser von 10 bis 80°C turbulent vermischt, daß ein Solubilisat mit 0,5 bis 6 Gew.-% β-Carotin entsteht, das gegebenenfalls auf die gewünschte Endkonzentration weiter verdünnt wird.

**2.** Verfahren gemäß Anspruch 1, dadurch gekennzeichnet, daß die Mischkammer ungefähr die Form eines T hat, in der das Wasser in einem Winkel von ca. 180° auf die heiße $\beta$-Carotinlösung trifft.

**3.** Verfahren gemäß Anspruch 1, dadurch gekennzeichnet, daß man einen nicht-ionogenen Emulgator verwendet, der einen HLB-Wert von 12 bis 16 aufweist.

**4.** Verfahren gemäß Anspruch 1, dadurch gekennzeichnet, daß man als nicht-ionogenen Emulgator ethoxilierte Monohydroxistearinsäure mit 15 Oxethyleneinheiten verwendet.

**5.** Verfahren gemäß Anspruch 1, dadurch gekennzeichnet, daß man die homogene Lösung mit dem Wasser in der Mischkammer bei einem auf 10 bis 50 bar erhöhten Druck turbulent vermischt.

**6.** Verfahren gemäß den Ansprüchen 1 bis 5, dadurch gekennzeichnet, daß man hierbei ein Antioxidans mitverwendet.

**7.** Verfahren gemäß Anspruch 1, dadurch gekennzeichnet, daß man als Antioxidans Butylhydroxitoluol, Butylhydroxianisol oder d,l-$\alpha$-Tocopherol in Mengen von 10 bis 20 Gew.-%, bezogen auf $\beta$-Carotin, verwendet.

**8.** Verfahren gemäß Anspruch 1 bis 6, dadurch gekennzeichnet, daß man ein Konservierungsmittel mitverwendet.

**9.** Verfahren gemäß Anspruch 8, dadurch gekennzeichnet, daß man als Konservierungsmittel Benzylalkohol verwendet.

**Claims**

**1.** A process for the continuous preparation of $\beta$-carotene solubilizates by briefly heating $\beta$-carotene together with an emulsifier to give a homogeneous solution, rapidly cooling the latter to below 100°C by adding water, and subsequently adjusting to the required final concentration of $\beta$-carotene, which comprises pumping a suspension, which has been preheated to from 20 to 80°C, of from 1 to 40 % by weight of $\beta$-carotene in an emulsifier through a heating coil located in a heat-transfer oil, where the solubilization mixture is at from 120 to 180°C and the residence time is from 10 to 300 seconds, and subjecting the homogeneous solution to turbulent mixing in a mixing chamber with an amount of water at from 10 to 80°C to result in a solubilizate which contains from 0.5 to 6 % by weight of $\beta$-carotene and is, if necessary, diluted to the required final concentration.

**2.** A process as claimed in claim 1, wherein the mixing chamber has approximately the shape of a T in which the water impinges on the hot $\beta$-carotene solution at an angle of about 180°.

**3.** A process as claimed in claim 1, wherein a nonionic emulsifier with an HLB of from 12 to 16 is used.

**4.** A process as claimed in claim 1, wherein the nonionic emulsifier used is ethoxylated monohydroxystearic acid with 15 oxyethylene units.

**5.** A process as claimed in claim 1, wherein the homogeneous solution is subjected to turbulent mixing with the water in the mixing chamber under a pressure which has been raised to 10 to 50 bar.

**6.** A process as claimed in any of claims 1 to 5, wherein an antioxidant is also used.

**7.** A process as claimed in claims 1, wherein the antioxidant is butylated hydroxytoluene, butylated hydroxyanisole or d,l-$\alpha$-tocopherol in an amount of from 10 to 20 % of the weight of $\beta$-carotene.

**8.** A process as claimed in any of claims 1 to 6, wherein a preservative is also used.

**9.** A process as claimed in claim 8, wherein benzyl alcohol is used as preservative.

6

**Revendications**

1. Procédé de préparation continue de produits de solubilisation du *β*-carotène par le chauffage de courte durée du *β*-carotène avec un émulsif jusqu'à solution, le refroidissement rapide de la solution homogène jusqu'en dessous de 100°C par l'addition d'eau et le réglage subséquent de la concentration finale en *β*-carotène souhaitée, caractérisé en ce que l'on pompe une suspension de 1 à 40% en poids de *β*-carotène dans un émulsif, préchauffée à 20-80°C, à travers un serpentin chauffant se trouvant dans une huile caloporteuse, où la température dans le mélange de solubilisation varie de 120 à 180°C et la durée de séjour fluctue de 10 à 300 secondes et on mélange de façon turbulente la solution homogène dans une chambre de mélange à des proportions d'eau de 10 à 80°C telles qu'un produit de solubilisation avec 0,5-6% en poids de *β*-carotène se forme, que l'on dilue éventuellement davantage jusqu'à la concentration finale souhaitée.

2. Procédé suivant la revendication 1, caractérisé en ce que la chambre de mélange possède à peu près la forme d'un T dans laquelle l'eau touche la solution de carotène chaude sous un angle d'environ 180°.

3. Procédé suivant la revendication 1, caractérisé en ce que l'on utilise un émulsif non ionogène, qui présente une valeur HLB de 12 à 16.

4. Procédé suivant la revendication 1, caractérisé en ce que, à titre d'émulsif non ionogène, on utilise l'acide monohydroxystéarique éthoxylé comportant 15 unités oxyéthylène.

5. Procédé suivant la revendication 1, caractérisé en ce que l'on mélange de manière turbulente la solution homogène à l'eau dans la chambre de mélange à une pression élevée jusqu'à 10 à 50 bars.

6. Procédé suivant l'une quelconque des revendications 1 à 5, caractérisé en ce que l'on utilise conjointement un antioxydant.

7. Procédé suivant la revendication 1, caractérisé en ce que l'on utilise, à titre d'agent antioxydant, le butylhydroxytoluène, le butylhydroxyanisole ou le d,l-*α*-tocophérol,en proportions de 10 à 20% en poids par rapport au *β*-carotène.

8. Procédé suivant l'une quelconque des revendications 1 à 6, caractérisé en ce que l'on utilise conjointement un conservateur.

9. Procédé suivant la revendication 8, caractérisé en ce que l'on utilise l'alcool benzylique à titre de conservateur.

Figur